# EUROPEAN PATENT SPECIFICATION

(11) **EP 1 545 365 B1**
(45) Date of publication and mention of the grant of the patent: **07.11.2007**
(21) Application number: 03734536.0
(22) Date of filing: 11.06.2003
(51) Int. Cl.: A61B 19/00

(54) **Medical device positioning system**
System zur Positionierung einer medizinischen Vorrichtung
Sytème de positionnement d'instruments médicaux

(30) Priority: 13.08.2002 US 64749
(43) Date of publication of application: 29.06.2005
(73) Proprietor: GENERAL ELECTRIC COMPANY, Schenectady, NY 12345 (US)
(72) Inventor: DARROW, Robert, David, Scotia, NY 12302 (US); DUMOULIN, Charles, Lucian, Ballston Lake, NY 12019 (US)
(74) Representative: Pedder, James Cuthbert
(86) International application number: PCT/US2003/018359
(87) International publication number: WO 2004/014246

(56) References cited:
- EP-A- 0 930 046
- US-A- 6 119 033
- US-A1- 2001 036 245
- US-B1- 6 400 980

## Description

### BACKGROUND OF THE INVENTION

This invention relates to systems for image guided interventional medical procedures in which a device is inserted into a body during imaging, and more particularly this invention relates to systems which assist in executing the diagnostic and interventional procedures such as assisting in the positioning of the device during the procedures.

Image guided medical or surgical procedures generally use an imaging technology such as magnetic resonance imaging (MRI) or compute tomography (CT) for generating images, either prior to the procedure or during the procedure, as a guide for a physician or operator of the system during the procedure. During interactive examinations/interventions with a medical device, such as a biopsy needle, whose guide is localized by a device tracking method, there is a need for a system to provide information to the operator to assist in precise and rapid placement of the guide. Device guides are positioned for the delivery of diagnostic or interventional devices, relative to a feature of interest such as a lesion. Proper placement of the medical device guide results in proper placement of the accompanying medical device, relative to a target.

During interactive examinations/interventions with a medical device, there is a need for a system to actively monitor the three-dimensional (3D) position of the device, and respond if the device has moved from its target position. Device motion is of importance for procedures where a therapy is applied to carefully selected and circumscribed areas. Device motion is of equal importance for a procedure where a tissue sample must be obtained from a precise location, such as a biopsy procedure.

Typically, in conventional tracking systems, the location of an interventional device is presented to a physician as a graphic symbol superimposed upon a diagnostic image. Due to time constraints, or the constraint of accumulated radiation dose, diagnostic images are acquired intermittently before tracking of the device commences, or are acquired at a much slower rate than the device is tracked. Consequently, if the subject or device moves after the acquisition of the diagnostic image, the representation of the device displayed to the physician may be mis-registered with respect to the diagnostic image.

US 6,119,033 discloses methods and apparatus for a medical imaging system-External sensors are used to estimate internal position of a medical device, in conjunction with an external image of a portion of a patient's anatomy and an internal image of e.g. a lesion.

What is needed is a system and method that overcomes the problems described above by monitoring and positioning the subject and device. In the event that motion is detected, it is desirable for a system and method to respond to, and correct for, the subject motion.

### BRIEF SUMMARY OF THE INVENTION

Aspects of the present invention are defined in the accompanying claims.

In a first aspect, a medical device positioning system for use during a medical procedure un a subject performed during imaging is provided. The system comprises a medical device adapted for internal use within the subject for performing the medical procedure and comprising one or more tracking elements for positioning internally in the subject, and an imaging device for acquiring image data of a region of interest within the subject. Additionally, the system includes a medical device monitoring subsystem for monitoring position of the medical device relative to a target region of interest within the subject by determining the position of the one or more tracking elements and for providing feedback to an interface unit when the position of the medical device deviates from the target region of interest.

### BRIEF DESCRIPTION OF THE DRAWINGS

The features and advantages of the present invention will become apparent from the following detailed description of the invention when read with the accompanying drawings in which:
Figure 1 is a perspective view of an exemplary medical imaging system in operation for monitoring and positioning the location of an invasive device in a subject to which embodiments of the present invention are applicable; and,
Figure 2 is an illustrative diagram of an acquired image employing embodiments of the present invention.

### DETAILED DESCRIPTION OF THE INVENTION

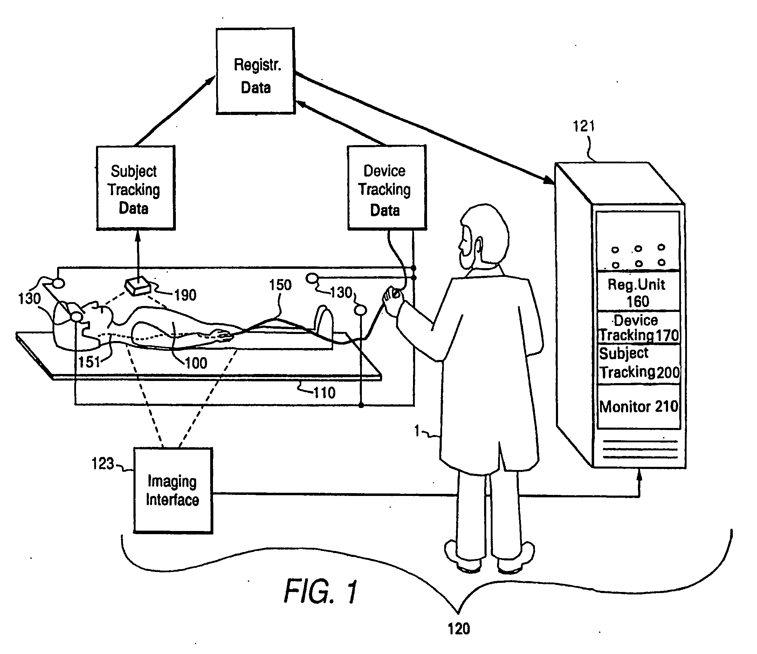

Referring to Figure 1, there is shown an imaging system to which embodiments of the present invention are applicable. In Fig. 1, a subject 100 on a support table 110 is placed in an imaging device 120, having imaging interface 123 and imaging processor 121, collectively referred to as imaging device 120. Imaging device 120 may be a magnetic resonance imaging (MRI) device, an X-Ray imaging device, a computed tomography (CT) scanner, a Positron Emission Tomography system or an ultrasound scanner, or any other conventional medical imaging device. An invasive device 150, shown in Fig 1 as a catheter, is inserted into subject 100, usually by physician 1. Device 150 may be a guide wire, a catheter, an endoscope, a laparoscope, a biopsy needle, a laser guide, a device guide, therapeutic laser or similar device.

Device 150 contains one or more element(s) 151, which may be easily tracked. For example, in an MR imaging device, it may be an RF coil that detects MR signals generated in subject 100. The element may also be an MR active substance such as a Fluorine compound that is tracked by MR Imaging. In the case of RF tracking, it may be an RF coil tracked by external RF coils 130.

Device tracking unit 170 determines the position of element 151 on device 150 relative to a fixed reference point, such as support table 110.

In the case of RF tracking, the location of device 150 is determined by employing several external detection devices, such as RF coils 130 around the perimeter of subject 100, and at least one internal coil of element 151 attached to device 150. The internal coil transmits RF energy that is received by the external RF coils 130 which are connected to device tracking unit 170. Device tracking unit 170 calculates the position of the internal coil over time. The transmission may be reversed such that external coils 130 transmit RF energy and internal coil of element 151 receives the transmitted RF energy.

In the case of MR tracking, element 151 detects nutation of magnetic resonance in a localized region around element 151. Device tracking unit 170 determines the location of element 151.

If more than one coil is used in element 151, determining the locations of all coils will also allow calculation of the orientation of device 150.

A position detection means 190, placed within the imaging device 120, measures position of one or more reference points of subject 100 over time. A reference image of the subject is acquired by the imaging device 120 at a time ti. The position of the reference points of subject 100 is monitored concurrently by position detection device 190. The image and corresponding subject location, and position are stored. In another embodiment, position detection means 190 may be comprised of light emitting diodes (LEDs) fixed to subject 100 and an optical detector capable of measuring distance to the LEDs at specified times. Also in another embodiment, position detection means 190 may comprise an ultrasonic tracking device that employs conventional ultrasound distance measurement techniques to determine the position of selected points on subject 100 at different times. In yet a further embodiment, position detection means 190 may comprise a mechanical tracking means such as a mechanical arm physically coupled to the subject to measure the width and height of a portion of the subject's anatomy.

Position information (subject tracking data) over time from motion detection means 190 is sent to a subject tracking unit 200 for processing. Subject tracking unit 200 computes translation and rotation movement of subject 100 from time tᵢ, the time of image acquisition, to time t_{d}, the time of device location measurement. This movement information is passed to a registration unit 160.

Registration unit 160 receives the reference image from imaging device 120 (shown as registration data), the net subject position and orientation change from subject tracking unit 200, and device 150 position and orientation from device (device tracking data) tracking unit 170. Registration unit 160 then translates and rotates the reference image to match the position and orientation of subject 100 at the time the location of device 150 location was measured. An image of device 150, or a graphic symbol of element 151 is synthesized by device tracking unit 170, or by registration unit 160. This image is superimposed upon the translated/rotated image of subject 100 at its absolute location and orientation to result in a registered image having both an image of subject 100 and device 150 correctly registered with each other.

Alternatively, registration unit 160 may transform the absolute location/orientation of device 150 in the reverse sense, then superimpose an image of device 150 at the transformed location/orientation on the reference image.

Subject tracking unit 200, registration unit 160 and device tracking unit 170 are shown as separate units for illustration reasons only. Generally, tracking, registration and device tracking information are sent for further processing by the imaging device (processor 121 of Figure 1). In embodiments of the present invention, processor 121 comprises subject tracking, registration and device tracking processing contained therein.

Proposed is a system where the operator is given active, precise and real-time guidance for positioning of a medical device guide. Such a system could be used for delivery of many different diagnostic and interventional devices. For example, it could be used to guide the placement of a therapeutic laser, or a biopsy needle guide.

Referring further to Figure 1, an embodiment of a medical device positioning system for use during a medical procedure on a subject while imaging the subject is provided herein. The system comprises a medical device, such as invasive device 150, and corresponding tracking device for example element 151, an imaging device 120 for acquiring images of the subject and medical device, and a medical device monitoring subsystem 210 for detecting movement of the device relative to a target region of interest on the subject. Monitoring subsystem 210 also provides feedback to an interface unit, such as interface 123, to assist operator 1 of the positioning system to position the medical device. The medical device 150 is adapted for internal use within the subject for performing the procedure. As used herein, the term medical procedure includes but is not limited to diagnostic procedures such as in vivo imaging, taking biopsies, surgical procedures and therapeutic procedures such as ablation, laser treatments, ultrasonic treatments, bracheatherapy and the like. Also, as used herein, "adapted to", "configured" and the like refer to mechanical or structural connections between elements to allow the elements to cooperate to provide a described effect; these terms also refer to operation capabilities of electrical elements such as analog or digital computers or application specific devices (such as an application specific integrated circuit (ASIC)) that are programmed to perform a sequel to provide an output in response to given input signals.

As described earlier, the medical imaging device 120 may be a magnetic resonance imaging (MRI) device, an X-Ray imaging device, a computed tomography (CT) scanner, a Positron Emission Tomography system or an ultrasound scanner, or any other conventional medical imaging device adapted to obtain medical diagnostic reference images. The device tracking system is a device tracking system capable of real-time localization in three dimensions, such as MR Tracking, RF Tracking and other methods known to one skilled in the art.

The device monitoring subsystem 210 is desirably integrated within processor 121 of Figure 1 and is adapted to monitor the position of a medical device relative to a target region of interest in the subject being imaged. In further embodiments of the device monitoring subsystem, the subsystem comprises device-specific configuration information from a configuration file which contains information relative to tracking method, such as position of rf coils, in device coordinates and further contains delivery information, such as position of exit hole and needle length for biopsy needle guide, in device coordinates. The monitoring subsystem is also coupled to interface 123 of Figure 1 so that an operator is able to mark the coordinates of a target position on reference images, either by recording the 3D position of target in system coordinates or by placing an indicator such as an icon on the images. Device monitoring subsystem 210 is adapted to receive input information from various sources and then converts the information to a common coordinate system. For example, target location marked by the operator on reference images, 3D coordinates of tracked locations on device guide or device specific tracking and delivery information.

Desirably, device monitoring subsystem 210 is also adapted to provide advisory feedback, desirably through interface 123 to provide feedback to the operator of the system regarding relative position of the medical device relative to the target region of interest. This capability allows an operator to target the two-dimensional (2D) or three-dimensional (3D) position adjusted for proper delivery of device and also to monitor a current 2D or 3D position of the device guide, in real-time. The advisory feedback is responsive to input from the monitoring subsystem and wherein the output is feedback to the operator for use in navigating or positioning of the device to reach the target location. The feedback may comprise audio instructions such as "rotate guide ten degrees clockwise", text output on a display screen (interface 123) such as "advance probe one inch" or visual output to show relative position of target and device guide on reference images.

The visual output of the advisory feedback could simply be unique icons on the reference image showing the target position and current position of the device guide, as described in the sample scenario below. When the icons coincide, then the guide has reached the desired position. The output could also be a much more sophisticated display. For example, the device configuration file could also include 3D coordinates of a wire-frame model of the device guide, in device coordinates. The 2D projection of the device guide could be superimposed on the 2D reference image to as an aid in positioning the guide. Additionally, the device configuration file could include the 3D coordinates of a wire-frame model of the medical device, and its 2D projection could be shown superimposed on the reference image. Supplementing this display might be additional device specific information, such as the projected needle track, or laser path.

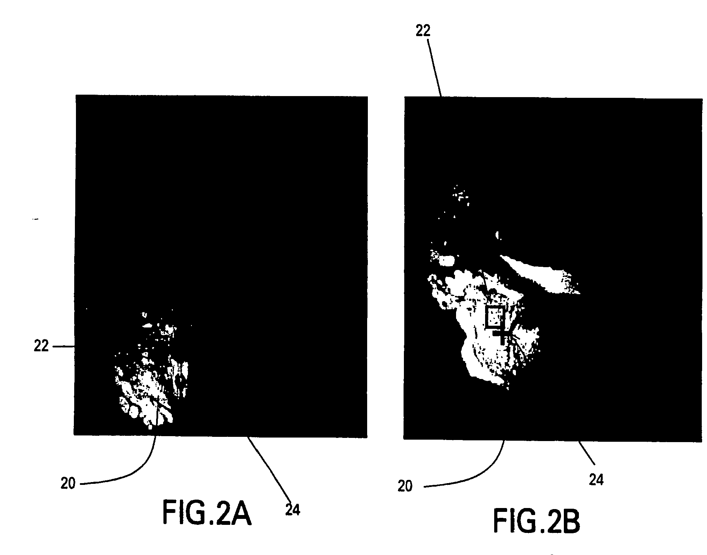

Referring to Figure 2 is an exemplary method in which a pair of 2D images is acquired wherein each image is in-plane with tracked locations of the medical device. The images may be acquired in the same plane, or desirably in two different planes (e.g. axial, sagittal or coronal) in order to be useful in positioning the device. Figure 2A shows an axial planar view of a region of interest 20 within a prostate, a target icon 22 and a sighting icon 24 and Figure 2B shows a second image, a sagittal planar view within the prostate, acquired at a later time and showing the relative position of the sighting icon and the target icon at a different view of region of interest 20. At the beginning of a medical procedure, the operator marks the location of the target on both of the acquired images with target icon 22. The result is a unique, stationary target icon 22 superimposed on the reference images. During the procedure to position the needle guide, sighting icon 24 appears on both reference images. In an embodiment for positioning the device, the operator uses the sighting icon and target icon to navigate the device. In this embodiment, the operator moves the device guide in such a way as to bring the sighting icon 24 closer to the target icon 22 in both planes. When the sighting icon 22 coincides with the target icon 24 in both planes, the device guide is properly positioned and the medical procedure (e.g. biopsy or therapy) can be performed. The operator may now insert the biopsy needle and perform the biopsy without further positioning. Additionally, projected needle paths or device outlines may be shown as separate visual outputs in order to be used in navigational applications.

In an embodiment for monitoring a device, device monitoring subsystem 210 of Figure 1 uses image processing techniques to mathematically compare the most currently acquired image and thereafter outputs to interface 123 an advisory message or output (e.g. audio or predetermined response) if comparison shows the device has moved more than an acceptable or predetermined threshold.

In further embodiments, the monitoring subsystem 210 is adapted to compute the recorded three-dimensional (3D) target position in system coordinates, the device coordinates of three tracking coils embedded in the guide, the device coordinates of the needle exit hole, the needle length and travel in device coordinates, and the real-time system coordinates of the three tracking coils in system coordinates. This information is desirably converted to a common coordinate system and combined to compare the 3D position of the target with the 3D position of the guide to offer advice on positioning the guide for a biopsy

In further embodiments, medical device monitoring subsystem 210 is responsive to either movement of the subject or movement of the medical device relative to a specified target region of interest within the subject. In one embodiment, the medical device subsystem 210 is adapted to respond to the movement with a predetermined response if the medical device position deviates by a specified distance from the target region of interest. For example, the monitoring subsystem 210 responds to motion of the medical device in pre-programmed fashion such as terminating therapy, acquiring new reference images, activating a device positioning subsystem to assist operator in repositioning device or alternatively activating advisory feedback.

Advisory feedback includes an output notification to operator, such as through interface 123 of Figure 1, that movement of the medical device relative to the target region of interest has occurred. For example, advisory feedback may include audio output such as "Device has moved. Laser has been shut down"; text output such as "Device has moved. Do you wish to reposition?"; and, visual output. In one embodiment, visual output may comprise as unique icons corresponding to the target and the device to showing the target position and current position of device. In a further embodiment visual output may show a two-dimensional (2D) projection of a wire-frame model of device or guide superimposed on the reference images. In yet a further embodiment, visual output may comprise a cartoon-like representation of the medical device superimposed on the reference images. Desirably, the visual output also shows device specific information on reference images, such as projected needle track, laser path, exit holes, needle length and similar device delivery information.

Also provided herein is a method for positioning a medical device comprising generating at least one image of a region of interest of a subject including a representation of a medical device superimposed in the image; monitoring a position of the medical device relative to a target region of interest within the subject; and, providing feedback to an interface upon detection of a change in position of the medical device relative to the target region. As described above, the feedback may include a first visual icon representing position of the device and a second visual icon representing the target region of interest, a text message, an audio advisory or a predetermined response. The predetermined response may include terminating therapy, activating the imaging device to acquire a new image or activating an advisory message to the interface unit. Desirably, the interface is adapted to respond to operator input of coordinates marking a target position of the medical device.

In further embodiments that are particular to MRI imaging applications, the monitoring subsystem 210 computes inputs from additional device-specific configuration information, such as information related to the tracking method, such as position of RF coils on device or guide, in device coordinates. Further, other inputs that would be useful in monitoring the device may be the static 3D coordinates of the centroid of the medical device or guide, when positioned at the target position which could be recorded when monitoring system activated or computed using a starting 3D position of device or guide from device tracking system, and tracked locations on device or guide. Alternatively, the real-time 3D position of tracked locations on device or guide from device tracking system could be used. In these embodiments, profiling beams (1D projections) are continuously acquired in axial, sagittal, and coronal planes and the profiling beams pass one of the following -- 3D position of centroid of medical device or guide or the 3D position of each of tracked location on device or guide. The monitoring subsystem thereafter mathematically compares most recently acquired profiling data with previously acquired profiling data and activates a response or feedback if the comparison shows device has moved significantly.

In a further MRI embodiment, where a MRI scanner is equipped with a MR Tracking system inputs may be from continuously acquired MR Tracking excitation data, such as from a body coil used for excitation or receiving excitation data with surface coil centered about target area, rather than tracking coil as is known in the art. The monitoring subsystem mathematically compares most recently acquired excitations with previously acquired excitations and activates a response or feedback if the comparison shows device has moved significantly. This embodiment allows simultaneous device tracking and motion detection. This is accomplished by using the same pulse sequence to excite spins for both functions. The MR tracking coils receive signals which can be used to determine device location, while the surface coil detects signals that are used to determine the global status and position of the region of interest.

While the preferred embodiments of the present invention have been shown and described herein, it will be obvious that such embodiments are provided by way of example only. Numerous variations, changes and substitutions will occur to those of skill in the art without departing from the invention herein. Accordingly, it is intended that the invention be limited only by the spirit and scope of the appended claims.

## Claims

1. A medical device positioning system for use during a medical procedure on a subject (100) performed during imaging, the system comprising:
a medical device (150) adapted for internal use within the subject (100) for performing the medical procedure and comprising one or more tracking elements (151) for positioning internally in the subject;
an imaging device (120) for acquiring image data of a region of interest within the subject during positioning of the medical device (150);
a medical device monitoring subsystem (210) for monitoring position of the medical device relative to a target region of interest within the subject by determining the position of the one or more tracking elements (151) internally within the subject and for providing feedback to an interface unit when the position of the medical device deviates from the target region of interest.

2. The system of claim 1 wherein the medical monitoring subsystem (210) is adapted to receive configuration information corresponding to the medical device and wherein the configuration information comprises at least one of three-dimensional (3D) co-ordinates of the device, tracking method information corresponding to the medical device, physical dimensions of the device and a model representation of the device.

3. The system of claim 1 wherein the medical device monitoring subsystem (210) is responsive to at least one of movement of the subject and movement of the medical device relative to the target region of interest within the subject.

4. The system of claim 3 wherein the medical device monitoring subsystem (210) responds to the movement with a predetermined response if the medical device position deviates by a specified distance from the target region of interest and wherein the predetermined response comprises at least one of terminating therapy, activating the imaging device to acquire a new image and activating an advisory message to the interface unit.

5. The system of claim 1, further comprising:
a tracking device (151) for tracking a location of the medical device; and,
a processor (121) coupled to the medical imaging device and the tracking device for generating images of the region of interest with a visual representation of the medical device superimposed on the images.

6. The system of claim 5 wherein the medical imaging device comprises at least one of a magnetic resonance imaging (MRI) scanner, a computed tomography (CT) scanner, a X-ray device, a Positron Emission Tomography (PET) system and an ultrasound scanner.

7. The system of claim 5 wherein the medical device (150) comprises at least one of a biopsy needle guide, an invasive probe, an ablation device, a laparoscope and a therapeutic laser.

## Patentansprüche

1. Positionierungsvorrichtung einer medizinischen Einrichtung zur Nutzung während einer medizinischen Prozedur , die während der Bildgebung an einem Objekt (100) durchgeführt wird, wobei die Vorrichtung aufweist:
eine medizinische Einrichtung (150), die zur internen Nutzung innerhalb des Objektes (100) zur Durchführung der medizinischen Prozedur ausgelegt ist und eine oder mehrere Tracking-Elemente (151) zur Positionierung im Inneren des Objektes aufweist;
eine Bildgebungseinrichtung (120) zum Akquirieren von Bilddaten in einem interessierenden Bereich innerhalb des Objektes während der Positionierung der medizinischen Einrichtung (150);
eine Monitoring-Untereinrichtung (210) der medizinischen Einrichtung zum Beobachten der Position der medizinischen Einrichtung relativ zu einem interessierenden Zielbereich innerhalb des Objekts durch internes Ermitteln der Position des einen oder der mehreren Tracking-Elemente (151) innerhalb des Objekts und zum Bereitstellen eines Feedbacks an eine Schnittstellen-Einrichtung, wenn die Position der medizinischen Einrichtung von dem interessierenden Zielbereich abweicht.

2. Vorrichtung nach Anspruch 1, worin die Monitoring-Untereinrichtung (210) der medizinischen Einrichtung dazu eingerichtet ist, Informationen zu erhalten, die zu der medizinischen Einrichtung gehören, und worin die Information der Konfiguration mindestens die dreidimensionalen (3D) Koordinaten der Einrichtung, die Informationen des Trackingverfahrens, die zu der medizinischen Einrichtung gehören, physikalische Dimensionen der Einrichtung und ein Modell umfassen, das die Einrichtung repräsentiert.

3. Vorrichtung nach Anspruch 1, worin die Monitoring-Untereinrichtung (210) der medizinischen Einrichtung auf mindestens eine der Bewegungen reagiert: die Bewegung des Objektes und die Bewegung der medizinischen Einrichtung relativ zu dem interessierenden Zielbereich innerhalb des Objektes.

4. Vorrichtung nach Anspruch 3, worin die Monitoring-Untereinrichtung (210) der medizinischen Einrichtung auf die Bewegung mit einer vorher ermittelten Antwort antwortet, wenn die Position der medizinischen Einrichtung durch eine spezifische Entfernung von dem interessierenden Zielbereich abweicht, und worin die vorher ermittelte Antwort mindestens eine der folgenden Anweisungen enthält: Beenden der Therapie, Aktivieren der Bildgebungseinrichtung, um ein neues Bild zu akquirieren, und Aktivieren einer Beratungsnachricht an die Schnittstellen-Einrichtung.

5. Vorrichtung nach Anspruch 1, die ferner aufweist:
eine Tracking-Einrichtung (151) zum Nachverfolgen eines Ortes der medizinischen Einrichtung; und
einen Prozessor (121), der mit der medizinischen Bildgebungseinrichtung und der Tracking-Einrichtung gekoppelt ist, zum Erzeugen von Bildern des interessierenden Bereichs mittels einer visuellen Darstellung der medizinischen Einrichtung, die den Bildern überlagert ist.

6. Vorrichtung nach Anspruch 5, worin die medizinische Bildgebungseinrichtung mindestens eine der folgenden Vorrichtungen aufweist: einen Magnetresonanz-Bildgebungs- (MRI)-Scanner, einen Computertomographie-(CT)-Scanner, eine Röntgenvorrichtung, eine Positronenemissionstomographie (PET)-Vorrichtung und einen Ultraschallscanner.

7. Vorrichtung nach Anspruch 5, worin die medizinische Einrichtung (150) mindestens eine der folgenden Vorrichtungen aufweist: Biotopsie-Nadelführung, eine invasive Sonde, eine Ablations-Einrichtung, ein Laparoskop und einen therapeutischen Laser.

## Revendications

1. Système de positionnement de dispositif médical destiné à être utilisé pendant une procédure médicale sur un sujet (100) exécutée pendant une visualisation par imagerie, le système comprenant :
un dispositif médical (150) prévu pour un emploi interne dans le sujet (100) pour effectuer la procédure médicale et comprenant un ou plusieurs éléments de suivi (151) pour le positionnement interne dans le sujet ;
un dispositif d'imagerie (120) pour acquérir des données d'images d'une région d'intérêt à l'intérieur du sujet pendant le positionnement du dispositif médical (150) ;
un sous-système de surveillance de dispositif médical (210) pour contrôler la position du dispositif médical par rapport à une région d'intérêt cible à l'intérieur du sujet en déterminant la position desdits un ou plusieurs éléments de suivi (151) à l'intérieur du sujet et pour fournir un retour à une unité d'interface quand la position du dispositif médical s'écarte de la région d'intérêt cible.

2. Système selon la revendication 1, dans lequel le sous-système de surveillance médical (210) est adapté pour recevoir de l'information de configuration correspondant au dispositif médical et dans lequel l'information de configuration comprend au moins un élément parmi des coordonnées tridimensionnelles (3D) du dispositif, des informations de méthode de suivi correspondant au dispositif médical, les dimensions physiques du dispositif et une représentation modélisée du dispositif.

3. Système selon la revendication 1, dans lequel le sous-système de surveillance de dispositif médical (210) réagit à au moins un paramètre parmi le mouvement du sujet et le mouvement du dispositif médical par rapport à la région d'intérêt cible à l'intérieur du sujet.

4. Système selon la revendication 3, dans lequel le sous-système de surveillance de dispositif médical (210) répond au mouvement avec une réponse prédéterminée si la position du dispositif médical s'écarte d'une distance spécifiée par rapport à la région d'intérêt cible et dans lequel la réponse prédéterminée comprend au moins un élément parmi l'arrêt de la thérapie, l'activation du dispositif d'imagerie pour prendre une nouvelle image et l'activation d'un message d'alerte en direction de l'unité d'interface.

5. Système selon la revendication 1, comprenant en outre :
un dispositif de suivi (151) pour suivre une position du dispositif médical ; et
un processeur (121) couplé au dispositif d'imagerie médicale et au dispositif de suivi pour produire des images de la région d'intérêt avec une représentation visuelle du dispositif médical superposée sur les images.

6. Système selon la revendication 5, dans lequel le dispositif d'imagerie médicale comprend au moins un système parmi un scanner d'imagerie par résonance magnétique (IRM), un scanner de tomographie informatisée (CT), un dispositif à rayons X, un système de tomographie par émission de positons (PET) et un scanner à ultrasons.

7. Système selon la revendication 5, dans lequel le dispositif médical (150) comprend au moins un dispositif parmi un guide aiguille de biopsie, une sonde effractive, un dispositif d'ablation, un laparoscope et un laser thérapeutique.
